# EUROPÄISCHE PATENTANMELDUNG

(11) **EP 1 520 854 A2**
(43) Veröffentlichungstag der Anmeldung: **06.04.2005**
(21) Anmeldenummer: 04029311.0
(22) Anmeldetag: 27.02.2003
(51) Int. Cl.: C07D 209/54, A61K 31/40

(54) **Aza-Spiroverbindungen zur Behandlung von Schmerzen**

(30) Priorität: 07.03.2002 DE 10210190
(62) Teilanmeldung aus: 03743342.2
(71) Anmelder: SCHWARZ PHARMA AG, 40789 Monheim (DE)
(72) Erfinder: Meese, Claus, 40789 Monheim (DE); Selve, Norma, 53842 Troisdorf (DE); Schmidt, Dirk, 40597 Düsseldorf (DE)

(57) **Zusammenfassung**

Die vorliegende Erfindung betrifft Azaspiroverbindungen und ihre Verwendung als Arzneimittel, insbesondere zur Behandlung von chronischem, chronisch-entzündlichem und/oder neuropathischem Schmerz. Eine zur Herstellung von Analgetika besonders geeignete Verbindung ist 2-Azaspiro[4.6]undecan-3-thion.

## Beschreibung

Neuropathische Schmerzen sind eine schwierig zu behandelnde Form von chronischen Schmerzen, die durch Verletzungen oder Erkrankungen des peripheren und/oder zentralen Nervensystems hervorgerufen werden und die nur schlecht auf traditionelle Analgetika ansprechen.

Auf Grund der Ähnlichkeiten in der Pathophysiologie von Epilepsie und neuropathischem Schmerz werden in jüngster Zeit zunehmend antikonvulsive Wirkstoffe zur Behandlung von neuropathischem Schmerz herangezogen. Ein Beispiel hierfür ist Gabapentin, das als Antiepileptikum bereits längere Zeit zugelassen ist, in jüngster Zeit aber auch zunehmend Bedeutung in der Behandlung von neuropathischem Schmerz bekommt (Tremont-Lukats, Drugs 60, 2000, 1029; Bock, Nervenarzt 72, 2001, 69).

Obwohl der Wirkmechanismus von Gabapentin noch nicht völlig aufgeklärt ist, hat Gabapentin durch seine Beeinflussung der glutaminergen/GABAergen Übertragung und der Beeinflussung von Calciumkanälen ein breites Wirkspektrum, dass von der Behandlung der Epilepsie über neuropathische und andere Schmerzzustände, wie z.B. Migräneschmerz (Block, Nervenarzt 72, 2001, 69) oder Muskel- und Skelettschmerz (EP 1 047 414), bis hin zur Behandlung von Depressionen (EP 552240), neurodegenerativen Erkrankungen (EP 446570), Angst- und Panikzuständen (EP 804 182) oder Manien (EP 825 857) reicht.

Ein Nachteil von Gabapentin ist die Bildung von toxischem Gabapentin-Lactam (2-Azaspiro [4.5]decan-3-one) bei Lagerung, und die damit verbundene Schwierigkeit, stabile Gabapentin-Formulierungen herzustellen.

In der WO 99/25683 werden eine große Zahl von in Position 4 substituierten Pyrrolidinon-Verbindungen, die auch Azaspiroverbindungen wie Gabapentin-Lactam umfassen, zur Behandlung von Erkrankungen vorgeschlagen, die mit erhöhten Glutamatspiegeln einhergehen, wie z.B. Epilepsie, Alzheimer, ALS oder Parkinson. In einem in-vitro Modell wird die Wirksamkeit des Gabapentin-Lactams in Ischämien und die Reduktion des Glutamat-Spiegels gezeigt. Ferner wird die neuroprotektive Wirkung von Gabapentin-Lactam in einem Ratten-Modell demonstriert. Auf Grund der Toxizität von Gabapentin-Lactam ist dies jedoch nicht zur Therapie am Menschen geeignet. Zudem gibt es in der WO 99/25683 keinen Hinweis darauf, dass die beanspruchten Pyrrolidone zur Behandlung von neuropathischem Schmerz geeignet sind.

In der DE 25 57 220 werden N-substituierte Gabapentin-Lactam-Derivate zur Behandlung von Epilepsie und zerebralen Störungen beschrieben. Die Verwendung bei Schmerzen wird nicht gelehrt.

Azaspiroverbindungen mit Aryl-Substituenten zur Behandlung von Schmerzen werden beschrieben in EP 337 547, EP 687 268, EP 880 528, EP 894 497, EP 906 315, EP 912 579, EP 929 554, EP 977 758 und EP 989 987. Es findet sich in diesen Dokumenten kein Hinweis darauf, dass auch Desaryl-Azaspiroverbindungen analgetisches Potential haben.

EP A 116 347 schlägt Amino-substituierte 1-Azaspiro[4.5.]decane und -undecane zur Behandlung von Schmerzen vor. 2-Azäspiroverbindungen werden nicht offenbart.

EP 310 321 beschreibt 2-Azaspiroverbindungen, in denen das Azaatom mit einer Stickstoff-haltigen Seitenkette substituiert ist. Die offenbarten Verbindungen werden als immunomodulatorisch beschrieben. Eine analgetische Wirkung wird nicht offenbart.

In der klinischen Praxis haben sich nur wenige Wirkstoffe bei der Behandlung von chronischen oder neuropathischen Schmerzen als wirksam und geeignet erwiesen, so dass in dieser Indikation weiterhin ein grosser Bedarf an innovativen Arzneimitteln besteht.

Ziel der vorliegenden Erfindung war es daher, alternative Arzneimittel zur Behandlung von Schmerz, insbesondere von chronischem, chronisch-entzündlichem und/oder neuropathischem Schmerz zur Verfügung zu stellen.

Dabei wurde überraschenderweise gefunden, dass von Gabapentin-Lactam abgeleitete Azaspiroverbindungen der allgemeinen Formel I eine stärkere analgetische Potenz als Gabapentin und Gabapentin-Lactam aufweisen und zugleich eine geringere Toxizität als Gabapentin-Lactam zeigen.

Die erfindungsgemäßen Azaspiroverbindungen, die zur therapeutischen Verwendung geeignet sind, haben die Formel I 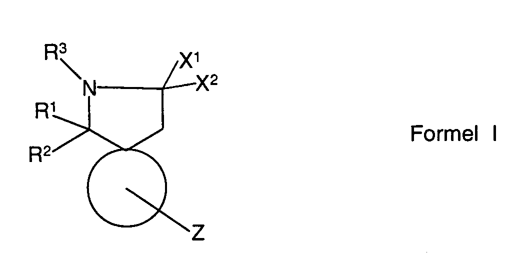 wobei
X¹ und X² entweder beide Wasserstoff sind oder gemeinsam eine Thioxo- oder Oximogruppe darstellen;
R¹ Wasserstoff ist und R² ausgewählt ist aus Wasserstoff, Hydroxy, Formyl, Carboxy, Halogen, Mercapto, Sulfonyl, Amino, Amido oder einer Gruppe -R⁷Q¹ oder wobei R¹ und R² gemeinsam eine Oxo- oder Thioxogruppe bilden;
R³ Wasserstoff, Hydroxy, Amino oder eine Gruppe -R⁸Q² ist;
Z ein mit dem ersten, heterocyclischen Ring über ein gemeinsames C-Atom verbundener gesättigter oder ungesättigter Ring ist, der inklusive dem Azaspiroatom 4-10 gliedrig ist, der neben Kohlenstoffatomen ein oder zwei ringbildende Heteroatome, ausgewählt aus O oder S aufweisen kann, und der unsubstituiert ist oder mit ein oder mehreren Substituenten ausgewählt aus Hydroxy, Formyl, Carboxy, Halogen, Mercapto, Sulfonyl, Amino, Amido oder einer Gruppe -R⁹Q³ substituiert ist,
R⁷ und R⁹ unabhängig voneinander C₁₋₅ Alkyl, C₃₋₆ Cycloalkyl, C₂₋₅ Alkenyl, C₂₋₅ Alkinyl, C₁₋₅ Alkoxy, C₁₋₅ Alkylcarbonyl, C₁₋₅ Alkoxycarbonyl, C₁₋₅ Alkylthio, C₁₋₅ Alkylamino, C₁₋₅ Alkylsulfinyl, C₁₋₅ Alkylsulfonyl, C₁₋₅ Alkylamino-C₁₋₅ Alkyl, C₁₋₅ Alkylthio-C₁₋₅ Alkyl oder C₁₋₅ Alkoxy-C₁₋₅ Alkyl sind; R⁸ ausgewählt ist aus C₁₋₅ Alkyl, C₃₋₆ Cycloalkyl, C₁₋₅ Alkoxy, C₁₋₅ Alkylcarbonyl, C₁₋₅ Alkoxycarbonyl, C₁₋₅ Alkylthio, C₁₋₅ Alkylamino, C₁₋₅ Alkylsulfinyl, C₁₋₅ Alkylsulfonyl, C₁₋₅ Alkylthio-C₁₋₅ Alkyl oder C₁₋₅ Alkoxy-C₁₋₅ Alkyl;
Q¹ und Q³ unabhängig voneinander Wasserstoff, Hydroxy, Formyl, Carboxy, Halogen, Mercapto, Sulfonyl, Amino oder Amido sind;
Q² ausgewählt ist aus Wasserstoff, Hydroxy, Formyl, Carboxy, Halogen, Mercapto, Sulfonyl oder Amido.

Die Azaspiroverbindungen können als freie Base oder als pharmazeutisch annehmbare Salze vorliegen und sind in beiden Formen Gegenstand der Erfindung.

Weiterhin können die Azaspiroverbindungen in Abhängigkeit von den Substituenten in verschiedenen tautomeren Formen vorliegen, die gegebenenfalls durch Salzbildung stabilisiert werden können. Auch diese Tautomeren und ihre Salze sind Gegenstand der Erfindung.

Als pharmazeutisch annehmbare Salze kommen alle bioverträglichen Salze in Frage, die die pharmakologischen Eigenschaften der Wirkstoffe weitgehend erhalten und keine unerwünschten toxischen Effekte auslösen. Beispiele hierfür sind insbesondere Additionssalze anorganischer oder organischer Säuren, wie z.B. Hydrogenchlorid, Hydrogenbromid, Essigsäure, Zitronensäure, Weinsäure, Oxalsäure, Fumarsäure, Äpfelsäure, Bernsteinsäure oder Methansulfonsäure.

Ferner ist dem Fachmann klar, dass in Abhängigkeit von den Substituenten Azaspiroverbindungen in optisch inaktiver oder aktiver Form existieren können. Daher werden reine Enantiomere ebenso wie Razemate oder optisch inaktive Verbindungen explizit zum Gegenstand der Erfindung gemacht.

Die obengenannten Begriffe sind im Sinne der vorliegenden Erfindung wie folgt zu verstehen:
Unter dem Ausdruck "C₁₋₅ Alkyl" wird in dieser Patentanmeldung ein Radikal einer gesättigten, aliphatischen Kohlenwasserstoffgruppe mit 1-5 C-Atomen verstanden, die verzweigt oder unverzweigt sein kann. Beispiele für C₁₋₅ Alkyle sind Methyl, Ethyl, n-Propyl, iso-Propyl, n-Butyl, iso-Butyl, s-butyl, t-butyl, n-Pentyl, iso-Pentyl, Neopentyl, t-Pentyl, 1-Methylbutyl, 2-Methylbutyl, 1-Ethylpropyl, 1,2-Dimethylpropyl.

Unter dem Ausdruck "C₂₋₅ Alkenyl" bzw. "C₂₋₅ Alkinyl" werden in dieser Patentanmeldung Radikale mit 2-5 C-Atomen verstanden, die sich von den Alkylen, wie oben definiert, dadurch entscheiden, dass sie mindestens eine Doppelbindung bzw. Dreifachbindung aufweisen.

Unter dem Ausdruck "C₃₋₆ Cycloalkyl" wird ein Radikal aus der Gruppe Cyclopropyl, Cyclobutyl, Cyclopentyl und Cyclohexyl verstanden.

Unter dem Ausdruck "Kohlenwasserstoffring" wird in dieser Patentanmeldung ein substituierter oder unsubstituierter Ring verstanden, dessen ringbildende Atome ausschließlich Kohlenstoffe sind und der somit frei von ringbildenden Heteroatomen ist.

Unter dem Ausdruck "C₁₋₅ Alkoxy" wird das Radikal -O-C₁₋₅ Alkyl verstanden.
Unter dem Ausdruck "C₁₋₅ Alkythio" wird das Radikal -S- C₁₋₅ Alkyl verstanden.
Unter dem Ausdruck "C₁₋₅ Alkylamino" wird das Radikal -NH- C₁₋₅ Alkyl verstanden
Unter dem Ausdruck "C₁₋₅ Alkylsulfinyl" wird das Radikal -S(O)- C₁₋₅ Alkyl verstanden.
Unter dem Ausdruck "C₁₋₅ Alkylsulfonyl" wird das Radikal -S(O₂)- C₁₋₅ Alkyl verstanden.
Unter dem Ausdruck "C₁₋₅ Alkylcarbonyl" wird das Radikal-C(O)-C₁₋₅ Alkyl verstanden
Unter dem Ausdruck "C₁₋₅ Alkoxycarbonyl" wird das Radikal -C(O)-O-C₁₋₅ Alkyl verstanden.
Unter "C₁₋₅ Alkylamino-C₁₋₅ Alkyl" wird die Gruppe C₁₋₅ Alkyl-NH-C₁₋₅ Alkyl verstanden
Unter "C₁₋₅ Alkylthio-C₁₋₅ Alkyl" wird die Gruppe C₁₋₅ Alkyl-S-C₁₋₅ Alkyl verstanden
Unter "C₁₋₅ Alkoxy-C₁₋₅ Alkyl" wird die Gruppe C₁₋₅ Alkyl-O-C₁₋₅ Alkyl verstanden

Unter dem Ausdruck "Halogen" wird ein Radikal aus der Gruppe F, Cl, Br, I verstanden. Unter dem Ausdruck "Thioxogruppe" wird die Gruppe =S verstanden.

Ein bevorzugter Gegenstand der Erfindung sind Verbindungen der allgemeinen Formel I zur medizinischen Verwendung, in denen der Ring Z inklusive Azaspiroatom ein 5-8-gliedriger Ring ist und besonders bevorzugt ein 5, 6 oder 7-gliedriger Ring ist.

Ein weiterer bevorzugter Gegenstand der Erfindung ist eine Verbindung der allgemeinen Formel I, in der Ring Z ein Kohlenwasserstoffring ist. In einer anderen Ausführungsform der Erfindung umfasst der Ring Z ein ringbildendes Sauerstoff oder Schwefelatom.

In einer weiteren bevorzugten Ausführungsform der Erfindung ist der Ring Z ein unsubstituierter Ring, besonders bevorzugt ein unsubstituierter Kohlenwasserstoffring, der inklusive Azaspiroatom 5, 6 oder 7 ringbildende C-Atome aufweist.

In einer anderen Ausführungsform der Erfindung ist der Ring Z ein 5, 6 oder 7-gliedriger Kohlenwasserstoffring, der mit einem Rest substituiert ist, der bevorzugt ausgewählt ist aus Hydroxy, Formyl, Carboxy, Halogen, Mercapto, Sulfonyl, Amino, Amido oder der Gruppe R⁹Q³, wobei R⁹ bevorzugt C₁₋₅ Alkyl ist und Q³ bevorzugt ausgewählt ist aus Wasserstoff, Hydroxy, Halogen, Amin oder Sulfonat.

In einer weiteren bevorzugten Ausführungsform ist der Ring Z gesättigt und stellt bevorzugt einen gesättigten Kohlenwasserstoffring dar, der besonders bevorzugt unsubstituiert ist.

In einer ganz besonders bevorzugten Ausführungsform der Erfindung ist der Ring Z inklusive Spiroatom Cyclopentan, Cyclohexan oder Cycloheptan.

In einer anderen bevorzugten Ausführungsform der Erfindung sind die Substituenten R¹ und R² der Verbindungen der Formel I jeweils Wasserstoff oder bilden beide zusammen eine Oxo- oder Thioxogruppe.

In einer anderen bevorzugten Ausführungsform der Erfindung ist der Substituent R³ C₁₋₅ Alkylcarbonyl, z.B. Methylcarbonyl, oder Wasserstoff.

In einer besonders bevorzugten Ausführungsform sind die Substituenten R¹ und R² jeweils Wasserstoff und R³ Wasserstoff oder Methylcarbonyl.

Eine andere bevorzugte Ausführungsform der Erfindung betrifft Azaspiroverbindungen der allgemeinen Formel I, wobei
X¹ und X² entweder beide Wasserstoff sind oder gemeinsam eine Thioxo- oder Oximogruppe darstellen;
R¹ Wasserstoff ist und R² ausgewählt ist aus Wasserstoff, Hydroxy, Formyl, Carboxy, Halogen, Mercapto, Sulfonyl, Amino, Amido oder einer Gruppe -R⁷Q¹ oder wobei R¹ und R² gemeinsam eine Oxo- oder Thioxogruppe bilden, wobei besonders bevorzugt ist, daß R¹ und R² jeweils Wasserstoff sind;
R³ Wasserstoff oder Methylcarbonyl ist,
Z ein mit dem ersten, heterocyclischen Ring über ein gemeinsames C-Atom verbundener gesättigter oder ungesättigter Kohlenwasserstoffring ist, der inklusive dem Azaspiroatom 5-7gliedrig ist, der frei von ringbildenden Heteroatomen ist und der unsubstituiert ist oder mit ein oder zwei Substituenten ausgewählt aus Hydroxy, Formyl, Carboxy, Halogen, Mercapto, Sulfonyl, Amino, Amido oder einer Gruppe -R⁹Q³ substituiert ist, wobei Z besonders bevorzugt gesättigt ist.
R⁷ und R⁹ unabhängig voneinander C₁₋₅ Alkyl, C₃₋₆ Cycloalkyl, C₂₋₅ Alkenyl, C₂₋₅ Alkinyl, C₁₋₅ Alkoxy, C₁₋₅ Alkylcarbonyl, C₁₋₅ Alkoxycarbonyl, C₁₋₅ Alkylthio, C₁₋₅ Alkylamino, C₁₋₅ Alkylsulfinyl, C₁₋₅ Alkylsulfonyl, C₁₋₅ Alkylamino-C₁₋₅ Alkyl, C₁₋₅ Alkylthio-C₁₋₅ Alkyl oder C₁₋₅ Alkoxy-C₁₋₅ Alkyl sind;
Q¹ und Q³ unabhängig voneinander Wasserstoff, Hydroxy, Formyl, Carboxy, Halogen, Mercapto, Sulfonyl, Amino oder Amido sind;
und wobei
die Azaspiroverbindungen als freie Base oder als pharmazeutisch annehmbare Salze vorliegen können.

Ganz besonders bevorzugte Azaspiroverbindungen der Formel I zur Verwendung als Arzneimittel sind
2-Azasprio[4.5]decan
2-Azaspiro[4.5]decan-3-thion
2-Azaspiro[4.4]nonan-3-thion
2-Azaspiro[4.6]undecan-3-thion
N-(2-Azaspiro[4.5]decan-3)-oxim
N-(2-Azaspiro[4.6]undecan)-3-oxim
1-(2-Azaspiro[4.5]dec-2yl)-ethanon

Ein weiterer Gegenstand der Erfindung sind pharmazeutische Zusammensetzungen, die eine Azaspiroverbindung der allgemeinen Formel I, wie oben beschrieben, sowie mindestens einen pharmazeutisch annehmbaren Hilfsstoff umfassen.

Dem Fachmann ist klar, dass die pharmazeutische Formulierung in Abhängigkeit vom beabsichtigten Applikationsweg unterschiedlich ausgestaltet sein kann. So kann die pharmazeutische Formulierung beispielsweise zur intravenösen, intramuskulären, intrakutanen, subkutanen, oralen, bukkalen, sublingualen, nasalen, transdermalen, inhalativen, rektalen oder intraperitonealen Verabreichung angepasst sein.

Entsprechende Formulierungen und hierfür geeignete pharmazeutische Träger bzw. Hilfsstoffe wie Füllstoffe, Sprengmittel, Bindemittel, Gleitmittel, Stabilisatoren, Aromastoffe, Antioxidanzien, Konservierungsmittel, Dispersions- oder Lösungsmittel, Puffer, Elektrolyte, sind dem Fachmann auf dem Gebiet der Pharmazeutik bekannt und sind beispielsweise in Standardwerken wie Sucker, Fuchs und Speiser ("Pharmazeutische Technologie", Georg Thieme Verlag, Stuttgart) beschrieben.

In einer bevorzugten Ausführungsform der Erfindung werden die die erfindungsgemäßen Verbindungen enthaltenden pharmazeutischen Zusammensetzungen oral verabreicht und können beispielsweise als Kapsel, Tablette, Pulver, Granulat, Dragee oder in flüssiger Form vorliegen.

Alternative pharmazeutische Zubereitungen können beispielsweise Infusions- oder Injektionslösungen, Öle, Suppositorien, Aerosole, Sprays, Mikrokapseln oder Mikropartikel sein.

Dabei kann die Formulierung als schnell freisetzende Darreichungsform ausgestaltet sein, wenn ein rascher Wirkeintritt gewünscht ist, z.B. bei akutem, bzw. akut durchbrechendem chronischen bzw. neuropathischen Schmerz. Entsprechende orale Formulierungen sind beispielsweise beschrieben in EP 159 237 oder EP 1 126 821.

Ist dagegen eine protrahierte Freisetzung erwünscht, bietet sich eine Formulierung mit retardierter Wirkstofffreisetzung an. Entsprechende orale Formulierungen sind ebenfalls aus dem Stand der Technik bekannt.

Die pharmazeutischen Formulierungen umfassen bevorzugt eine Verbindung der allgemeinen Formel I 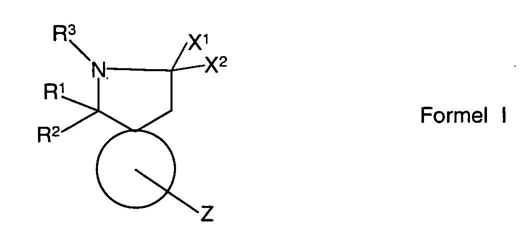 worin R¹, R², R³, X¹, X² und Z die weiter oben angegebene Bedeutung haben.

Ein bevorzugter Gegenstand der Erfindung sind pharmazeutische Zusammensetzungen umfassend Azaspiroverbindungen der allgemeinen Formel I, in der der Ring Z der Azaspiroverbindung ein unsubstituierter Ring und/oder ein gesättigter Kohlenwasserstoffring ist, der inklusive Azaspiroatom 5, 6 oder 7 ringbildende C-Atome aufweist.

In einer ganz besonders bevorzugten Ausführungsform der Erfindung umfasst die pharmazeutische Formulierung eine Azaspiroverbindung der allgemeinen Formel I, in der der Ring Z inklusive Spiroatom ein gesättigter, unsubstitutierter Kohlenwasserstoffring und insbesondere Cyclopentan, Cyclohexan oder Cycloheptan ist.

In einer anderen bevorzugten Ausführungsform der Erfindung umfasst die pharmazeutische Formulierung eine Azaspiroverbindung der allgemeinen Formel I, wobei die Substituenten R¹ und R² jeweils Wasserstoff sind oder beide zusammen eine Oxo- oder Thioxogruppe bilden.

In einer besonders bevorzugten Ausführungsform sind die Substituenten R¹ und R² jeweils Wasserstoff und R³ ist Wasserstoff oder eine Methylcarbonylgruppe.

In einer anderen bevorzugten Ausführungsform der Erfindung bilden X¹ und X² gemeinsam eine Thioxo- oder Oximogruppe, R¹ und R² sind jeweils Wasserstoff, R³ ist Wasserstoff oder Methylcarbonyl und Z ein gesättigter Köhlenwasserstoffring, der besonders bevorzugt unsubstituiert ist und bevorzugt 5-8 ring bildende Atome aufweist.

Ganz besonders bevorzugte pharmazeutische Zusammensetzungen umfassen Azaspiroverbindungen der Formel I, die ausgewählt sind aus
2-Azaspiro[4.5]decan-3-thion
2-Azaspiro[4.4]nonan-3-thion
2-Azaspiro[4.6]undecan-3-thion
2-Azasprio[4.5]decan
N-(2-Azaspiro[4.5]decan-3)-oxim
N-(2-Azaspiro[4.6]undecan-3)-oxim
1-(2-Azaspiro[4.5]dec-2yl)-ethanon
und deren pharmazeutisch annehmbare Salze, sowie einen pharmazeutisch annehmbaren Träger oder Hilfsstoff.

Ein weiterer Gegenstand der Erfindung sind Verkaufspackungen, umfassend mindestens eine pharmazeutische Formulierung, wie oben beschrieben, sowie eine Anleitung zu deren Verwendung. Eine solche Verkaufspackung kann auch weitere Arzneimittel enthalten. Zum Beispiel könnte die Verkaufspackung zusätzlich ein weiteres Analgetikum, ein Sedativum, ein Ergotamin-Derivat, ein Antiemetikum, ein Antiphlogistikum oder ein Antidepressivum enthalten.

Überraschenderweise zeigen die erfindungsgemäßen Verbindungen in pharmakologischen Vergleichstests eine starke Wirksamkeit im Formalintest, einem in-vivo Test für die Voraussage der potentiellen Wirksamkeit einer Substanz bei der Behandlung von chronischem, bzw. chronisch-entzündlichem und/oder neuropathischem Schmerz (Tjolsen and Herle, Handbook Exp. Pharmacol. Vol 130, Ed: Dickensen & Besson, Springer Verlag 1997, Seite 6).

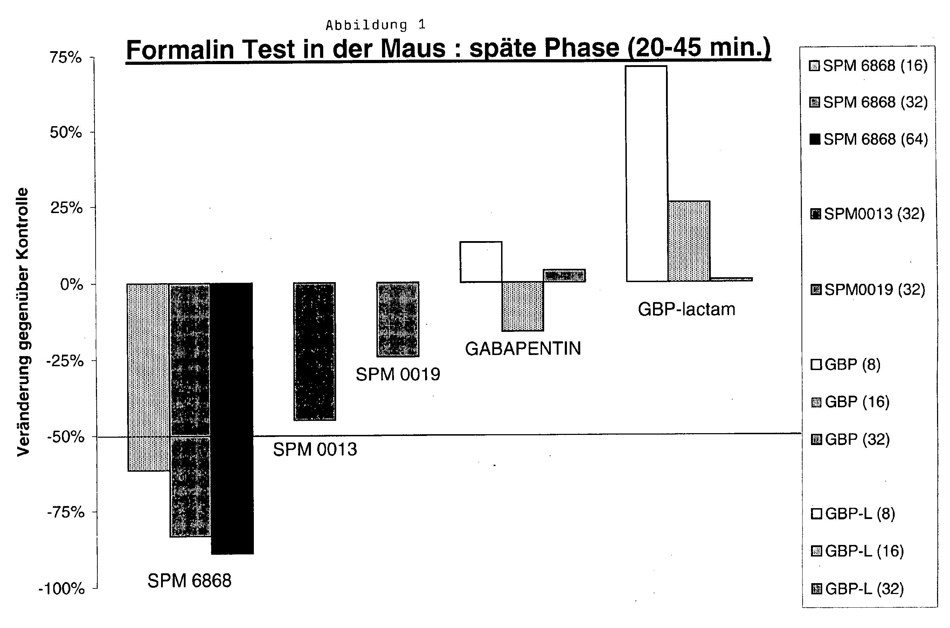

Abbildung 1 und Tabelle 1 zeigen jeweils die Reaktionen von Versuchstieren (je 10 Mäuse) 20 - 45 Minuten nach intraperitonealer Gabe ausgewählter Verbindungen. Dabei wurden jeweils als maximale Dosierung Konzentrationen der Azaspiroverbindungen gewählt, die um ca. einen Faktor 2 unterhalb der toxischen Dosis liegen, die zuvor im IRWIN-Test (Irwin, Psychopharmacologia 13 (1968) 222) bestimmt wurde.

Dabei haben die in Abbildung 1 verwendeten Abkürzungen die folgende Bedeutung: SPM 6868 entspricht 2-Azaspiro[4.6]undecan-3-thion, SPM 0013 entspricht 2-Azaspiro[4.5]decan-3-thion, SPM 0019 entspricht 2-Azaspiro[4.5]decan. GBP bedeutet Gabapentin. GBP-L bedeutet Gabapentin-Lactam. In der Legende wird jeweils in Klammern hinter der Substanzbezeichnung die Dosierung der Substanzen in mg/kg Körpergewicht angegeben.

Wie aus Abbildung 1 hervorgeht, haben die erfindungsgemäßen Verbindungen überraschenderweise im Formalintest eine signifikant höhere Potenz als Gabapentin und Gabapentin-Lactam, die als Vergleich eingesetzt wurden.

Darüberhinaus zeigten auch die beiden Verbindungen N-(2-Azaspiro[4.5]decan-3)-oxim (SPM 6850) und N-(2-Azaspiro[4.6]undecan-3)-oxim (SPM 6873) im oben beschriebenen Formalintest nach 30-45 Minuten eine mittlere Reduktion der Schmerzreaktion um 44% bzw. 36,5%, wie Tabelle 1 zeigt:

**Tabelle 1**

| Verbindung | Dosierung (mg/kg) | mittlere Abweichung der Schmerzreduktion gegenüber Kontrolle (%) (x Minute nach Formalingabe) | | |
|---|---|---|---|---|
| | | 20-25' | 30-35' | 40-45' |
| SPM 6868 | | | | |
| Meßreihe 1 | 64 | - 89 | - 67 | - 83 |
| (n=10) | 32 | - 83 | 0 | - 24 |
| | 16 | - 62 | - 29 | - 49 |
| Meßreihe 2 | 64 | - 100 | - 98 | - 93 |
| (n-10) | 16 | - 42 | - 30 | - 31 |
| | 4 | - 53 | - 7 | -16 |
| SPM 0013 (n=10) | 32 | - 99 | - 32 | - 4 |
| SPM 0019 (n=10) | 32 | - 67 | - 21 | - 26 |
| SPM 6850 (n=10) | 32 | (+) | - 50 | - 38 |
| SPM 6873 (n=10) | 16 | (+) | - 35 | - 38 |
| GBP-L | | | | |
| Meßreihe 1 | 32 | - 64 | - 15 | - 46 |
| (n=10) | 16 | - 74 | - 40 | - 41 |
| | 8 | - 58 | - 18 | - 15 |
| Meßreihe 2 | 32 | (+) | - 10 | nb |
| (n=10) | 16 | (+) | (+) | nb |
| | 8 | (+) | (+) | nb |
| Morphium* | 8 | - 87 | - 95 | - 88 |

| | | | | |
|---|---|---|---|---|
| (+): Verstärkung der Schmerzreaktion | | | | |
| *) Mittelwert aus 9 Meßreihen | | | | |
| nb = nicht bestimmt | | | | |

Schließlich erwiesen sich die oberhalb der maximal tolerablen Dosen einsetzenden Nebenwirkungen der erfindungsgemäßen Verbindungen (Sedierung, Tremor, Hypothermie) als weit weniger schwer als diejenigen des GPL, bei dem eine signifikante Letalitätsrate zu beobachten war.

Die erfindungsgemäß für die Therapie geeigneten Azaspiroverbindungen sind somit besonders zur Behandlung von Schmerzen, insbesondere von chronischen, chronisch-entzündlichen und/oder neuropathischen Schmerzen geeignet.

Ein Gegenstand der Erfindung ist daher die Verwendung einer Azaspiroverbindung der allgemeinen Formel II 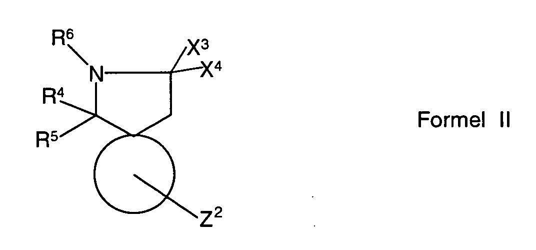 wobei
X³ und X⁴ entweder beide Wasserstoff sind oder gemeinsam eine Thioxo- oder Oximogruppe darstellen;
R⁴ Wasserstoff ist und R⁵ ausgewählt ist aus Hydroxy, Formyl, Carboxy, Halogen, Mercapto, Sulfonyl, Amino, Amido oder einer Gruppe -R¹⁰Q⁴ oder wobei R⁴ und R⁵ gemeinsam eine Oxo- oder Thioxogruppe bilden;
R⁶ Wasserstoff, Hydroxy, Amino oder eine Gruppe -R¹¹Q⁵ ist;
Z² ein mit dem ersten, heterocyclischen Ring über ein gemeinsames C-Atom verbundener gesättigter oder ungesättigter Ring ist, der inklusive Azaspiroatom 4-10 gliedrig ist, der neben Kohlenstoffatomen ein oder zwei ringbildende Heteroatome, ausgewählt aus N, O oder S aufweisen kann, und der unsubstituiert ist oder mit ein oder mehreren Substituenten ausgewählt aus Wasserstoff, Hydroxy, Formyl, Carboxy, Halogen, Mercapto, Sulfonyl, Amino, Amido oder einer Gruppe -R¹²Q⁶ substituiert ist;
R¹⁰, R¹¹ und R¹² unabhängig voneinander C₁₋₅ Alkyl, C₃-C₆ Cycloalkyl, C₂₋₅ Alkenyl, C₂₋₅ Alkinyl, C₁₋₅ Alkoxy, C₁₋₅ Alkylcarbonyl, C₁₋₅ Alkoxycarbonyl, C₁₋₅ Alkylthio, C₁₋₅ Alkylamino, C₁₋₅ Alkylsulfinyl, C₁₋₅ Alkylsulfonyl, C₁₋₅ Alkylamino-C₁₋₅ Alkyl, C₁₋₅ Alkylthio-C₁₋₅ Alkyl oder C₁₋₅ Alkoxy-C₁₋₅ Alkyl sind;
Q⁴, Q⁵ und Q⁶ unabhängig voneinander Wasserstoff, Hydroxy, Formyl, Carboxy, Halogen, Mercapto, Sulfonyl, Amino oder Amido sind;
sowie möglicher Tautomere und/oder pharmazeutisch annehmbarer Salze
zur Herstellung eines Arzneimittels zur Behandlung von Schmerzen, insbesondere von chronischen, chronisch-entzündlichen und/oder neuropathischen Schmerzen.

Die die erfindungsgemäßen Azaspiroverbindungen enthaltenden Arzneimittel können grundsätzlich zur Behandlung verschiedener Schmerzformen, wie z.B. Migräneschmerzen, Skelett- und Muskelschmerzen etc. verwendet werden. Besonders geeignet sind die die erfindungsgemäßen Azaspiroverbindungen enthaltenden Arzneimittel aber zur Behandlung von chronischen, chronisch-entzündlichen und/oder neuropathischen Schmerzen.

Neuropathischer Schmerz ist eine komplexe Erkrankung, die oft als Folgeerkrankung von Verletzungen, Infektionen, metabolischen Störungen und degenerativen Erkrankungen des Nervensystems auftreten kann. Beispiele für neuropathische Schmerzsyndrome sind beispielsweise Phantomschmerzen, postherpetische Neuralgien nach Gürtelrose, schmerzhafte diabetische Neuropathien, komplexe regionale Schmerzsyndrome, verschiedene Arten von Krebsschmerzen, neuropathische Schmerzen in Zusammenhang mit Multipler Sklerose oder mit Verletzungen größerer Nervenbahnen, dem Rückenmark oder dem Stammhirn.

Ein bevorzugter Gegenstand der Erfindung ist die Verwendung einer Verbindung der allgemeinen Formel II, in der der Ring Z² inklusive Azaspiroatom ein 5-8-gliedriger und besonders bevorzugt ein 5, 6 oder 7-gliedriger Ring ist, zur Herstellung eines Schmerzmittels.

In einer weiteren bevorzugten Ausführungsform der Erfindung wird zur Herstellung des Schmerzmittels eine Verbindung der allgemeinen Formel II verwendet, in der der Ring Z² ein Kohlenwasserstoffring ist. In einer anderen Ausführungsform der Erfindung umfasst der Ring Z² ein ringbildendes Sauerstoff oder Schwefelatom.

In einer weiteren bevorzugten Ausführungsform der Erfindung wird zur Herstellung des Analgetikums eine Azaspiroverbindung der Formel II verwendet, in der der Ring Z² ein unsubstituierter Ring, besonders bevorzugt ein unsubstituierter Kohlenwasserstoffring ist, der inklusive Azaspiroatom 5, 6 oder 7 ringbildende C-Atome aufweist.

In einer anderen Ausführungsform der Erfindung ist der Ring Z² ein 5, 6 oder 7-gliedriger Kohlenwasserstoffring, der mit einem Rest substituiert ist, der bevorzugt ausgewählt ist aus Hydroxy, Formyl, Carboxyl, Halogen, Mercapto, Sulfonyl, Amino, Amido oder der Gruppe -R¹²Q⁶, wobei R¹² bevorzugt C₁₋₅ Alkyl ist und Q⁶ bevorzugt ausgewählt ist aus Wasserstoff, Hydroxy, Halogen, Amino oder Sulfonyl.

In einer weiteren bevorzugten Ausführungsform ist der Ring Z² der im Analgetikum verwendeten Azaspiroverbindung gesättigt und stellt bevorzugt einen gesättigten Kohlenwasserstoffring dar, der besonders bevorzugt unsubstituiert ist.

In einer ganz besonders bevorzugten Ausführungsform der Erfindung ist der Ring Z² des analgetischen Wirkstoffs der Formel II inklusive Spiroatom Cyclopentan, Cyclohexan oder Cycloheptan.

In einer anderen bevorzugten Ausführungsform der Erfindung wird zur Herstellung des Schmerzmittels eine Azaspiroverbindung der allgemeinen Formel II verwendet, wobei die Substituenten R⁴ und R⁵ jeweils Wasserstoff sind oder beide zusammen eine Oxo- oder Thioxogruppe bilden.

In einer anderen bevorzugten Ausführungsform der Erfindung ist der Substituent R⁶ Methylcarbonyl oder Wasserstoff.

In einer besonders bevorzugten Ausführungsform sind die Substituenten R⁴, R⁵ und R⁶ jeweils Wasserstoff.

In einer Ausführungsform der Erfindung wird zur Herstellung des Arzneimittels zur Behandlung von Schmerzen, insbesondere von chronischen, chronisch-entzündlichen und/oder neuropathischen Schmerzen eine Azaspirobehandlung der allgemeinen Formel II verwendet, wobei
X³ und X⁴ entweder beide Wasserstoff sind oder gemeinsam eine Thioxo- oder Oximogruppe darstellen;
R⁴ Wasserstoff ist und R⁵ ausgewählt ist aus Hydroxy, Formyl, Carboxy, Halogen, Mercapto, Sulfonyl, Amino, Amido oder einer Gruppe -R¹⁰Q⁴ oder wobei R⁴ und R⁵ gemeinsam eine Oxo- oder Thioxogruppe bilden, wobei besonders bevorzugt wird, das R⁴ und R⁵ beide Wasserstoff sind,
R⁶ Wasserstoff oder Methylcarbonyl, wobei R⁶ besonders bevorzugt Wasserstoff ist;
Z² ein mit dem ersten, heterocyclischen Ring über ein gemeinsames C-Atom verbundener gesättigter oder ungesättigter Kohlenwasserstoffring ist, der inklusive Azaspiroatom 5-7gliedrig ist, der frei von ringbildenden Heteroatomen ist und der unsubstituiert ist oder mit ein oder zwei Substituenten ausgewählt aus Wasserstoff, Hydroxy, Formyl, Carboxy, Halogen, Mercapto, Sulfonyl, Amino, Amido oder einer Gruppe -R¹²Q⁶ substituiert ist, wobei Z² besonders bevorzugt ein gesättigter Kohlenwasserstoffring ist.
R¹⁰ und R¹² unabhängig voneinander C₁₋₅ Alkyl, C₃-C₆ Cycloalkyl, C₂₋₅ Alkenyl, C₂₋₅ Alkinyl, C₁₋₅ Alkoxy, C₁₋₅ Alkylcarbonyl, C₁₋₅ Alkoxycarbonyl, C₁₋₅ Alkylthio, C₁₋₅ Alkylamino, C₁₋₅ Alkylsulfinyl, C₁₋₅ Alkylsulfonyl, C₁₋₅ Alkylamino-C₁₋₅ Alkyl,
C₁₋₅ Alkylthio-C₁₋₅ Alkyl oder C₁₋₅ Alkoxy-C₁₋₅ Alkyl sind;
Q⁴ und Q⁶ unabhängig voneinander Wasserstoff, Hydroxy, Formyl, Carboxy, Halogen, Mercapto, Sulfonyl, Amino oder Amido sind und wobei die Azaspiroverbindung gegebenenfalls als freie Base oder als pharmazeutisch annehmbares Salz vorliegen kann.

Zur Herstellung eines Schmerzmittels besonders bevorzugt werden solche Azaspiroverbindungen, die im Formalintest, wie in Beispiel 8 beschrieben, in mindestens einem, bevorzugt in mindestens zwei der Testzeiträume (20-25', 30-35', 40-45' nach Formalingabe) eine mittlere Abweichung der Schmerzreaktion von wenigstens - 40 %, bevorzugt mindestens - 50 % oder - 60 % bewirken.

Unter dem Begriff "mittlere Abweichung der Schmerzreaktion" wird in dieser Anmeldung die relative Abweichung verstanden, die sich ergibt, wenn die gemittelte Zeit der Schmerzreaktion von 10 mit Wirkstoffen behandelten Tieren einer Versuchsreihe, wie in Ausführungsbeispiel 8 beschrieben, in einem definierten Zeitraum (20-25', 30-35' oder 40-45' nach Formalininjektion) mit der gemittelten Zeit der Schmerzreaktion von 10 mit Vehikel behandelten Kontrolltieren verglichen wird. Eine Reduktion der Schmerzreaktion wird dabei in negativen Prozentzahlen angegeben.

Ganz besonders bevorzugt werden Azaspiroverbindungen der Formel II, die ausgewählt sind aus
2-Azaspiro[4.5]decan-3-thion
2-Azaspiro[4.4]nonan-3-thion
2-Azaspiro[4.6]undecan-3-thion
2-Azaspiro[4.5]decan
N-(2-Azaspiro[4.5]decan-3)-oxim
N-(2-Azaspiro[4.6]undecan-3)-oxim
1-(2-Azaspiro[4.5Jdec-2yl)-ethanon
und deren pharmazeutisch annehmbare Salze zur Herstellung eines Arzneimittels zur Behandlung von Schmerzen, insbesondere von chronischen, chronisch-entzündlichen und/oder neuropathischen Schmerzen, verwendet.

Schließlich betrifft die Erfindung eine neue Azaspiroverbindung, die ausgewählt ist aus
2-Azaspiro[4.5]decan-3-thion
2-Azaspiro[4.4]nonan-3-thion
2-Azaspiro[4.6]undecan-3-thion
2-Azaspiro[4.5]decan
N-(2-Azaspiro[4.5]decan-3)-oxim
N-(2-Azaspiro[4.6]undecan-3)-oxim
1-(2-Azaspiro[4.5]dec-2yl)-ethanon

Die Erfindung wird durch die nachfolgenden Beispiele näher beschrieben.

### 1. Herstellung von 2-Azaspiro[4.5]decan-3-thion

In einem Einhalskolben wurden 1,01 g (6,6 mmol) Gabapentinlactam, 1,6 g (4,0 mmol) Lawessons' Reagenz und 20 ml Toluol vorgelegt. Die gelbe Lösung wurde unter Rückfluß (130°C) 20 Stunden gerührt. Nach dem Abkühlen wurde über eine Kieselgelsäule abfiltriert (Essigsäure als Laufmittel). Das Lösungsmittel wurde abgezogen.
Der gelbe Feststoff wurde noch einmal über eine Kieselgelsäule aufgereinigt und nachfolgend eine weitere Säulentrennung mit Aluminiumoxyd durchgeführt. Das Lösemittel wurde abgezogen und der Rückstand aus 40 ml Diisopropylether bei 4°C umkristallisiert.
Die Ausbeute betrug 25 % d.Th. (278 mg)
Der Schmelzpunkt beträgt 121,9°C (Büchi B-545, 1°C/min)
NMR (CDCl₃): 204.64; 60.19; 55.44; 42.18; 36.03; 25.35; 22.90.

### 2. Herstellung von 2-Azaspiro[4.4]nonan-3-thion

In einem Einhalskolben wurden 3,93 g (28,2 mmol) 2-Azaspiro[4.4]nonan-3-on, 6,5 g Lawessons Reagenz und 100 ml Toluol vorgelegt. Das Reaktionsgemisch wurde 20 Stunden unter Rückfluß erhitzt. Dabei entstand eine gelbe Lösung
Das Lösemittel wurde am Rotationsverdampfer abgezogen.
Der Rückstand wurde über eine Aluminiumoxidsäule chromatographiert (Laufmittel: Dichlormethan).
Das Laufmittel wurde abdestilliert und der zurückbleibende Feststoff in 100 ml Diisopropylether und 15 ml Dichlormethan mit Aktivkohle aufgekocht. Die Aktivkohle wurde heiß abfiltriert und das Filtrat auf +4°C gekühlt. Dabei fielen weiße Kristalle aus.
   Die Ausbeute betrug 1,14 g (26 % d.Th.).
NMR (CDCl₃): 205.24; 60.50; 55.66; 49.42; 37.60; 23.73.

### 3. Herstellung von 2-Azaspiro[4.6]undecan-3-thion

In einem Einhalskolben wurden 4,18 g (25 mmol) 2-Azaspiro[4.6]undecan-3-on, 5,8 g (14,3 mmol) Lawessons' Reagenz und 125 ml Toluol vorgelegt und sodann unter Rückfluß für 18 Stunden refluxiert. Die abgekühlte Lösung wurde über Aluminiumoxid (neutral) abfiltiert. Das Filtrat wurde einrotiert und der Rückstand aus 60 ml Diisopropylether umkristallisiert.
Die Ausbeute betrug 1,2 g (26,2 % d.Th.).
Der Schmelzpunkt betrug 132,3°C (Büchi B-545, 1 °C/min)
NMR: 205.05; 61.59; 57.32; 45.83; 39.50; 28.68; 23.26.

### 4. Herstellung von 2-Azaspiro[4.5]decan

In einem 100 ml-Dreihalskolben mit Magnetrührer, Tropftrichter und Rückflußkühler wurden 28 ml 1,0 M Lithiumaluminiumhydrid in THF (28,0 mmol) vorgelegt. Dazu wurde unter Rühren und Abkühlen auf 0°C-10°C eine Lösung von 5,01 g (32,7 mmol) Gabapentinlactam (2-Aza-spiro[4.5]decan-3-on) in 30 ml THF zugetropft. Diese Reaktion war sehr exotherm und fand unter H₂-Entwicklung statt.
Nach Beendigung des Zutropfens wurde noch 4 Stunden unter Rückfluß erhitzt.
Anschließend wurde das Reaktionsgemisch auf 0°C abgekühlt und mit einer Mischung von 2 ml Wasser und 2 ml THF versetzt, um das überschüssige LiAlH₄ zu vernichten. Nach der endgültigen Zersetzung wurden noch weitere 10 ml THF dazugegeben, um die Lösung zu verdünnen. Das Reaktionsgemisch wurde mit 10 g NaSO₄ versetzt und noch 10 Minuten gerührt.
Das Natriumsulfat wurde abfiltriert und dreimal mit jeweils 20 ml THF gewaschen. Das Lösemittel wurde abgezogen. Zurück blieb Amin als klares, farbloses Öl.
Die Ausbeute betrug 4,06 g (89,2 % d.Th.).
NMR (CDCl₃): 59.03; 46.28; 42.98; 38.72; 37.01; 25.66; 23.78.

### 5. Herstellung von N-(2-Azaspiro[4.5]decan-3)-oxim

1,69 g des wie in Beispiel 1 beschrieben hergestellten 2-Azaspiro[5.5.]decan-3-thion wurden in 200 ml Ethanol gelöst. Nach zugabe von 25 ml 50%iger Hydroxylaminlösung in Wasser wurde der Ansatz über Nacht bei Raumtemperatur gerührt.
Der Ansatz wurde vollständig einrotiert. Der ölige Rückstand wurde in 10 ml Wasser aufgenommen und kristallisierte nach kurzer Zeit durch. Das Produkt wurde abgesaugt und im Vakuum bei Raumtemperatur getrocknet.
Die Ausbeute des farblosen Produkts betrug 63,10 % d.Th. (1,06 g)
Der Schmelzpunkt wurde mit 156,9°C bestimmt (Büchi B-545, 1°C/min).
NMR (CDCl₃): 158.30; 55.59; 40.98; 38.86; 36.09; 25.93; 23.27.

### 6. Herstellung von N-(2-Azaspiro[4.6]undecan-3)-oxim

550 mg des wie unter Beispiel 3 beschrieben hergestellten 2-Azaspiro[4.6]undecan-3-thion wurden in 60 ml Ethanol gelöst. Nach Zugabe von 15 ml 50%ige Hydroxylaminlösung in Wasser wurde der Ansatz über Nacht bei Raumtemperatur gerührt.
Der Ansatz wurde vollständig einrotiert. Der ölige Rückstand wurde in 10 ml Wasser aufgenommen und kristallisierte nach kurzer Zeit durch. Das Produkt wurde abgesaugt und im Vakuum bei Raumtemperatur getrocknet.
Die Ausbeute des farblosen Produkts betrug 91,1 % d.Th (460 mg)
Der Schmelzpunkt wurde mit 140.8 °C bestimmt (Büchi B-545, 1 °C/min).
NMR(CDCL₃):158.38; 56.90; 44.36; 40.87; 39.01; 29.21; 23.61; 23.25.

### 7. Herstellung von 1-(2-Azaspiro[4.5]dec-2yl)-ethanon

1,98 g des wie in Beispiel 4 beschrieben hergestellten 2-Azapiro[4.5.]decans wurden in 10 ml Dichlormethan verdünnt. Unter Argon wurden dazu 2.5 ml Triethylamin zugegeben und die Lösung auf 0°C abgekühlt. Unter Rühren wird eine Lösung von 1.2 ml Acetylchlorid in 10 ml Dichlormethan zugetropft. Danach wird 2 Stunden bei 0°C und eine Stunde bei Raumtemperatur gerührt.
Zum Reaktionsgemisch werden 20 ml Wasser gegeben und die Phasen getrennt. Die organische gelbe Phase wird mit 20 ml 1 M HCl und mit 20 ml Wasser gewaschen. Die wässrigen Phasen werden mit Dichlormethan gewaschen. Die organischen Phasen werden über Natriumsulfat getrocknet, das Natriumsulfat abfiltriert und das lösungsmittel abdestilliert. Der Rückstand wird zweimal über eine Kieselgelsäule (Laufmittel: Triethylamin/Essigester 1:9) chromatographiert.
Die Ausbeute betrug 100% d.Th (2,54 g).
NMR (CDCl₃): 169.28; 58.03; 55.49; 45.74; 43.87; 35.17; 25.98; 23.20; 22.01.

### 8. In vivo-Test zur Bestimmung der analgetischen Wirksamkeit der Azaspiroverbindungen

Der Test wurde durchgeführt wie durch Wheeler-Aceto (Psychopharmacology, 104, 1991, 35) beschrieben.
NMRI-Mäuse mit einem Gewicht von 20-25 g wurden unter kontrollierten Bedingungen (22 ± 2°C, 40-70 % Luftfeuchtigkeit) gehalten. 25 µl einer 5 % Formcarbonyllösung wurde in die Hinterpfote injiziert und nachfolgend zu definierten Zeitpunkten (20, 30, 40 Minuten) für jeweils 5 Minuten die Zeit des Leckens der Pfoten gemessen.
Die höchstmögliche einsetzbare, nicht-toxische Konzentration der jeweiligen Testsubstanzen wurde zunächst im IRWIN-Test bestimmt (Irwin, Psychopharmacologia 13, 1968, 222).
Die Testsubstanzen wurden in physiologischer Kochsalzlösung mit 0,5 % Natriumcarboxymethylzellulose gelöst und jeweils in 3 Dosierungen gemessen, die 10 Minuten vor Formalingabe intraperitoneal appliziert wurden. Als Vergleichswert diente eine Vehikelkontrolle (10 ml/kg).
Der Test wurde verblindet mit je 10 Mäusen pro Versuchsreihe durchgeführt. Die Auswertung erfolgte durch einen Vergleich der behandelten Tiere mit Vehikelkontrollen zu drei verschiedenen Zeitpunkten. Hierzu wurde jeweils für die 10 Tiere einer Versuchsreihe pro Zeitraum ein Mittelwert für die Schmerzreaktion ermittelt und dann die relative Abweichung der mit Wirkstoff behandelten Tiere von den Kontrolltieren für jeden der drei verschiedenen Zeiträume bestimmt. Eine mittlere Reduktion der Schmerzreaktion für die behandelten Tiere um 50 % ergibt sich demzufolge, wenn für einen definierten Zeitraum (z.B. 30-35' nach Formalininjektion) die Zeit des Leckens der Pfote (gemittelt über die 10 Versuchstiere) gegenüber unbehandelten Tieren um 50 % reduziert ist. Die statistische Signifikanz wurde mittels Mann-Whitney U-Test ermittelt.

## Patentansprüche

1. Azaspiroverbindung der allgemeinen Formel I 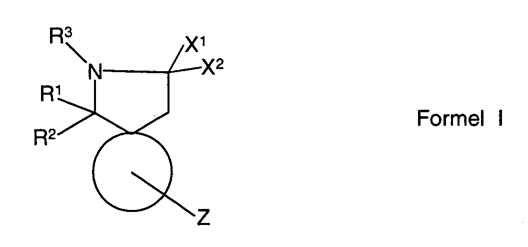 wobei X¹ und X² entweder beide Wasserstoff sind oder gemeinsam eine Thioxooder Oximogruppe darstellen;
R¹ Wasserstoff ist und R² ausgewählt ist aus Wasserstoff, Hydroxy, Formyl, Carboxy, Halogen, Mercapto, Sulfonyl, Amino, Amido oder einer Gruppe -R⁷Q¹ oder wobei R¹ und R² gemeinsam eine Oxo- oder Thioxogruppe bilden
R³ Wasserstoff, Hydroxy, Amino oder eine Gruppe -R⁸Q² ist,
Z ein mit dem ersten, heterocyclischen Ring über ein gemeinsames C-Atom verbundener gesättigter oder ungesättigter Ring ist, der inklusive dem Azaspiroatom 4-10 gliedrig ist, der neben Kohlenstoffatomen ein oder zwei ringbildende Heteroatome, ausgewählt aus O oder S aufweisen kann, und der unsubstituiert ist oder mit ein oder mehreren Substituenten ausgewählt aus Wasserstoff, Hydroxy, Formyl, Carboxy, Halogen, Mercapto, Sulfonyl, Amino, Amido oder einer Gruppe -R⁹Q³ substituiert ist,
R⁷ und R⁹ unabhängig voneinander C₁₋₅ Alkyl, C₃₋₆ Cycloalkyl, C₂₋₅ Alkenyl, C₂₋₅ Alkinyl, C₁₋₅ Alkoxy, C₁₋₅ Alkylcarbonyl, C₁₋₅ Alkoxycarbonyl, C₁₋₅ Alkylthio, C₁₋₅ Alkylamino, C₁₋₅ Alkylsulfinyl, C₁₋₅ Alkylsulfonyl, C₁₋₅ Alkylamino-C₁₋₅ Alkyl, C₁₋₅ Alkylthio-C₁₋₅ Alkyl oder C₁₋₅ Alkoxy-C₁₋₅ Alkyl sind,
R⁸ ausgewählt ist aus C₁₋₅ Alkyl, C₃₋₆ Cycloalkyl, C₁₋₅ Alkoxy, C₁₋₅ Alkylcarbonyl, C₁₋₅ Alkoxycarbonyl, C₁₋₅ Alkylthio, C₁₋₅ Alkylamino, C₁₋₅ Alkylsulfinyl, C₁₋₅ Alkylsulfonyl, C₁₋₅ Alkylthio-C₁-₅ Alkyl oder C₁₋₅ Alkoxy-C₁₋₅ Alkyl,
Q¹ und Q³ unabhängig voneinander Wasserstoff, Hydroxy, Formyl, Carboxy, Halogen, Mercapto, Sulfonyl, Amino oder Amido sind,
Q² ausgewählt ist aus Wasserstoff, Hydroxy, Formyl, Carboxy, Halogen, Mercapto, Sulfonyl oder Amido,
sowie deren mögliche Tautomere und/oder pharmazeutisch annehmbare Salze, als Arzneimittel.

2. Azaspiroverbindung als Arzneimittel nach Anspruch 1, wobei R³ Wasserstoff oder C₁₋₅ Alkylcarbonyl ist.

3. Azaspiroverbindung als Arzneimittel nach einem der vorhergehenden Ansprüche, wobei Z ein 5, 6 oder 7 gliedriger Ring ist.

4. Azaspiroverbindung als Arzneimittel nach einem der vorhergehenden Ansprüche, wobei Z ein Ring ist, der gesättigt ist.

5. Azaspiroverbindung als Arzneimittel nach einem der vorhergenden Ansrpüche, wobei Z ein unsubstituierter Ring ist.

6. Azaspiroverbindung als Arzneimittel nach einem der vorhergehenden Ansprüche, wobei Z ein Kohlenwasserstoffring ist.

7. Azaspiroverbindung als Arzneimittel nach einem der vorhergehenden Ansprüche, wobei Z Cyclopentan, Cyclohexan oder Cycloheptan ist.

8. Azaspiroverbindung als Arzneimittel nach einem der vorhergehenden Ansprüche, wobei R¹ und R² jeweils Wasserstoff sind oder zusammen eine Oxo- oder Thioxogruppe bilden.

9. Azaspiroverbindung als Arzneimittel nach einem der vorhergehenden Ansprüche, wobei R¹ und R² jeweils Wasserstoff sind und R³ Wasserstoff oder Methylcarbonyl ist.

10. Azaspiroverbindung als Arzneimittel, wobei die Azaspiroverbindung ausgewählt ist aus der Gruppe
2-Azaspiro[4,5]decan-3-thion
2-Azaspiro[4,4]nonan-3-thion
2-Azaspiro[4,6]undecan-3-thion
2-Azaspiro[4,5]decan
N-(2-Azaspiro[4.5]decan-3)-oxim
N-(2-Azaspiro[4.6]undecan-3)-oxim.

11. Pharmazeutische Zusammensetzung umfassend eine Azaspiroverbindung nach einer der vorhergehenden Ansprüche und mindestens ein pharmazeutisches Hilfsmittel.

12. Verwendung einer Azaspiroverbindung der allgemeinen Formel II 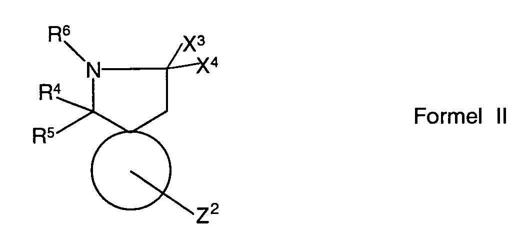 wobei X³ und X⁴ entweder beide Wasserstoff sind oder gemeinsam eine Thioxo- oder Oximogruppe darstellen;
R⁴, R⁵ entweder unabhängig voneinander Wasserstoff, Hydroxy, Formyl, Carboxy, Halogen, Mercapto, Sulfonyl, Amino, Amido oder eine Gruppe -R¹⁰ Q⁴ sind oder gemeinsam eine Oxo- oder Thioxogruppe bilden;
R⁶ Wasserstoff, Hydroxy, Amino oder eine Gruppe -R¹¹Q⁵ ist
Z² ein mit dem ersten, heterocyclischen Ring über ein gemeinsames C-Atom verbundener gesättigter oder ungesättigter Ring ist, der inklusive dem Azaspiroatom 4-10 gliedrig ist, der neben Kohlenstoffatomen ein oder zwei ringbildende Heteroatome, ausgewählt aus N, O oder S aufweisen kann, und der unsubstituiert ist oder mit ein oder mehreren Substituenten ausgewählt aus Wasserstoff, Hydroxy, Formyl, Carboxy, Halogen, Mercapto, Sulfonyl, Amino, Amido oder einer Gruppe -R¹²Q⁶ substituiert ist;
R¹⁰, R¹¹ und R¹² unabhängig voneinander C₁₋₅ Alkyl, C₃-C₆ Cycloalkyl, C₂₋₅ Alkenyl, C₂₋₅ Alkinyl, C₁₋₅ Alkoxy, C₁₋₅ Alkylcarbonyl, C₁₋₅ Alkoxycarbonyl, C₁₋₅ Alkylthio, C₁₋₅ Alkylamino, C₁₋₅ Alkylsulfinyl, C₁₋₅ Alkylsulfonyl, C₁₋₅ Alkylamino-C₁₋₅ Alkyl, C₁₋₅ Alkylthio-C₁₋₅ Alkyl oder C₁₋₅ Alkoxy-C₁₋₅ Alkyl sind;
Q⁴, Q⁵ und Q⁶ unabhängig voneinander Wasserstoff, Hydroxy, Formyl, Carboxy, Halogen, Mercapto, Sulfonyl, Amino oder Amido sind,
oder eines möglichen Tautomers und/oder pharmazeutisch annehmbaren Salzes zur Herstellung eines Arzneimittels zur Behandlung von Schmerzen.

13. Verwendung nach Anspruch 11, wobei R⁶ Wasserstoff oder C₁₋₅ Alkylcarbonyl ist.

14. Verwendung nach einem der vorhergehenden Ansprüche, wobei Z² ein 5, 6 oder 7 gliedriger Ring ist.

15. Verwendung nach einem der vorhergehenden Ansprüche, wobei Z² ein Ring ist, der gesättigt ist.

16. Verwendung nach einem der vorhergehenden Ansprüche, wobei Z² ein unsubstituierter Ring ist.

17. Verwendung nach einem der vorhergehenden Ansprüche, wobei Z² ein Kohlenwasserstoffring ist.

18. Verwendung nach einem der vorhergehenden Ansprüche, wobei Z² Cyclopentan, Cyclohexan oder Cycloheptan ist.

19. Verwendung nach einem der vorhergehenden Ansprüche, wobei R⁴ und R⁵ jeweils Wasserstoff sind oder zusammen eine Oxo- oder Thioxogruppe bilden.

20. Verwendung nach einem der vorhergehenden Ansprüche, wobei R⁴ und R⁵ jeweils Wasserstoff sind und R⁶ Wasserstoff oder Methylcarbonyl ist.

21. Verwendung nach Anspruch 12, wobei die Azaspiroverbindung ausgewählt ist aus der Gruppe.
2-Azaspiro[4,5]decan-3-thion
2-Azaspiro[4,4]nonan-3-thion
2-Azaspiro[4,6]undecan-3-thion
2-Azaspiro[4,5]decan
N-(2-Azaspiro[4.5]decan-3)-oxim
N-(2-Azaspiro[4.6]undecan)-3-oxim

22. Verwendung nach einem der vorhergehenden Ansprüche, wobei der Schmerz chronischer, chronisch-entzündlicher und/oder neuropathischer Schmerz ist.

23. Azaspiroverbindung ausgewählt aus der Gruppe
2-Azaspiro[4,5]decan-3-thion
2-Azaspiro[4,4]nonan-3-thion
2-Azaspiro[4,6]undecan-3-thion
2-Azaspiro[4,5]decan
N-(2-Azaspiro[4.5]decan-3)-oxim
N-(2-Azaspiro[4.6]undecan)-3-oxim
1-(2-Azaspiro[4.5]dec-2yl)-ethanon
